# EUROPEAN PATENT APPLICATION

(11) **EP 1 352 952 A1**
(43) Date of publication of application: **15.10.2003**
(21) Application number: 02729529.4
(22) Date of filing: 09.01.2002
(51) Int. Cl.: C12M 1/34, C12Q 1/02, G01N 33/15, G01N 33/48, G01N 33/50, G01N 27/28, G01N 27/416

(54) **DEVICE FOR MEASURING EXTRACELLULAR POTENTIAL, METHOD OF MEASURING EXTRACELLULAR POTENTIAL BY USING THE SAME AND APPARATUS FOR QUICKLY SCREENING DRUG PROVIDED THEREWITH**

(30) Priority: 09.01.2001 JP 2001001980; 05.06.2001 JP 2001170341
(71) Applicant: MATSUSHITA ELECTRIC INDUSTRIAL CO., LTD., Kadoma-shi, Osaka 571-8501 (JP)
(72) Inventor: OKA, Hiroaki, Hirakata-shi, Osaka 573-1194 (JP); OGAWA, Ryuta, Moriguchi-shi, Osaka 574-0031 (JP); YUKIMASA, Tetsuo, Hirakata-shi, Osaka 573-1122 (JP); SUGIHARA, Hirokazu, Katano-shi, Osaka 576-0054 (JP); KONISHI, Satoshi, Otsu-shi, Shiga 520-0112 (JP); JIKOH, Hideyasu, Hirakata-shi, Osaka 573-0005 (JP); OZAKI, Nobuhiko, Ikoma-shi, Nara 630-0239 (JP); EMOTO, Fumiaki, Hirakata-shi, Osaka 573-0051 (JP)
(74) Representative: UEXKÜLL & STOLBERG
(86) International application number: JP0200061
(87) International publication number: WO02055653

(57) **Abstract**

An extracellular potential measuring device capable of simply and highly reliably detecting a change in a minute electrical signal emitted by a biological sample, and an extracellular potential measuring method using the device, and an apparatus comprising the device, are provided. The extracellular potential measuring device measures an extracellular potential with great precision and a high output. The device comprises at least one well comprising means for holding a cell provided on a substrate, a measuring electrode for detecting an electrical signal of each of the at least one well, and a reference electrode. The cell holding means comprises at least one depression provided within the well, and has a throughhole at a bottom surface thereof, the throughhole being linked to means for suctioning the cell.

## Description

### TECHNICAL FIELD

The present invention relates to a device for measuring an extracellular potential, which is used for drug screening to simply and quickly perform electrophysiological evaluation of drug screening of biological samples, particularly cells. The present invention also relates to a method for measuring a physicochemical change (particularly, an electrochemical change) in a biological sample (particularly, a cell), a reaction system for use in this method, and an apparatus comprising this reaction system. More specifically, the present invention relates to a method for evaluating an electrochemical change generated by a cell, which is related to a macro activity of the entire cell (particularly, noise is compared using a voltage change as an index), and a quick drug screening apparatus using the method.

### BACKGROUND ART

Drug screening has been conducted using the electrical activity of a cell as an index. Conventionally, the electrical activity of a cell-is measured by a patch clamp method, a method employing a fluorescent pigment or a light emitting indicator, or the like.

In a patch clamp technique, a small portion (patch) of cell membrane is attached to a tip portion of a micropipette with a microelectrode probe, and is used to electrically record ion transport through a single ion channel protein. The patch clamp technique is one of a few number of cell biological techniques which can be used to investigate the function of a single protein in real time (see, e.g., Molecular Biology of the Cell, 3rd Ed., Garland Publishing, Inc., New York, 1994, Japanese Version, translation supervised by Keiko Nakamura et al., pp. 181-182, 1995, Kyoikusha). Further, there is a method in which a light emitting indicator or a fluorescent pigment, which emits light in response to a change in the concentration of a particular ion, is combined with a state-of-the-art image processing method (e.g., a fluorescent image of a cell is picked up by a CCD camera or the like to monitor intracellular ion transport) so as to measure the electrical activity of a cell.

In a method of using a microelectrode probe, such as a patch clamp (intracellular recording method), an electrophysiological measurement apparatus comprising a microelectrode probe and a dedicated control apparatus is employed to measure the amount of electricity passing through an ion channel of a subject cell, thereby making it possible to determine the duration, the timing or the number of times of opening or closing of the ion channel of the cell. Thus, a single ion channel can be analyzed.

In an alternative method using a fluorescent pigment, the ion channel activity of an entire cell is determined by, representatively, measuring the overall amount of ions entering the cell by a fluorescent measurement method.

The patch clamp technique requires special techniques for preparation, manipulation and the like of a micropipette, and much time for measuring one sample. Therefore, the patch clamp technique is not suitable for screening a large quantity of candidate compounds for a drug at high speed. The fluorescence measurement technique can screen a large quantity of candidate compounds for a drug at high speed. However, the fluorescence measurement technique requires a step of staining a cell. During measurement, pigments cause high background noise, and the fluorescence intensity decreases with time, resulting in poor signal to noise ratio (S/N).

Another technique for observing an electrochemical change in a biological sample is disclosed in JP No. 2949845, USP No. 5810725, USP No. 5563067, Japanese Laid-Open Publication No. 9-827318, WO 01/25769 A2, USP No. 5187069, WO 98/54294, WO 99/66329, NO 99/31503, and the like, in which a substrate provided with multiple electrodes is employed.

JP No. 2949845, USP No. 5810725, USP No. 5563067, and Japanese Laid-Open Publication No. 9-827318 disclose an integrated multiple electrode comprising microelectrodes prepared by photolithography on a glass substrate and capable of measuring electrical changes in cells, and a measurement system using the same.

WO 01/25769 A2 discloses a substrate in which an insulating substrate provided with throughholes and a biological sample, such as a cell containing an ion channel, is placed on the throughholes so that a gigaseal is provided on the surface of the insulating substrate including the cell; a reference electrode and a measuring electrode, which are provided in two respective domains separated by the gigaseal, can be used to measure electric current generated by ions passing through an ion channel of the cell.

USP No. 5187069 discloses a device capable of monitoring the growth of cells by culturing the cells on electrodes and measuring impedance changes.

WO 98/54294 discloses a device in which cells are adhered onto a planar electrode and an electrical signal therefrom is measured.

WO 99/66329 discloses a device for observing the activity of cells on a porous material by measuring resistance or impedance changes, and an assay using the same.

WO 99/31503 discloses a method in which a substrate provided with throughholes is employed, patch clamps are established by trapping cells with the throughholes, and changes in electric current are measured.

The above-described conventional techniques are characterized in that the electrical activity of cells is determined on a planar electrode, and minute throughholes are provided in an insulating substrate so that patch clamps are formed between cells and the substrate, whereby electric current generated by ions passing through an ion channel can be monitored. However, when a planar electrode is employed, a signal emitted from a biological sample leaks into solution so that the sensitivity of the measurement is disadvantageously reduced. In the method in which small throughholes are provided in an insulating substrate so that patch clamps are formed with cells and the holes, the possibility of forming patch clamps is low. Moreover, the cell membrane must be destroyed or physically injured to form patch clamp, so that it is not possible to measure the activity of intact cells. Patch clamp formation techniques also have a difficulty in adjusting suctioning pressure for a biological sample. Multiple channels would impractically require multiple pressure adjusting mechanisms. In these regards, conventional planar electrodes are suitable for quick drug screening but have poor sensitivity. The insulating substrate having small throughholes is not suitable for quick drug screening. For similar reasons, an automated patch clamping robot requires much time for sample processing and is not suitable for quick drug screening.

As described above, a number of problems remain in the field of quick drug screening.

There is a demand for an extracellular potential measuring method with which data having substantially the same quality as that obtained by a patch clamp method can be obtained and which can be performed simply, quickly and automatically, as with the fluorescent pigment method. There is also a demand for a device and apparatus capable of directly and quickly monitoring the activity of a cell, without using any pigment.

### DISCLOSURE OF THE INVENTION

An object of the present invention is to improve conventional electrophysiological measurement apparatuses so as to solve the above-described problems.

The present invention relates to a device for measuring an extracellular potential, comprising at least one well comprising means for holding a cell provided on a substrate, a measuring electrode for detecting an electrical signal of each of the at least one well, and a reference electrode.

Preferably, the cell holding means comprises at least one depression provided within the well, and has a throughhole at a bottom surface thereof, the throughhole being linked to means for suctioning the cell.

Preferably, the measuring electrode is disposed within a space, the space being in communication with the well via the throughhole.

Preferably, the cell is tightly held by an opening of the depression.

Preferably, the opening of the depression has a diameter of 10 to 50 µm and the throughhole has a diameter of about 2 to 10 µm.

Preferably, the substrate is made of a material selected from the group consisting of a silicon wafer, Teflon, polystyrene and polycarbonate.

Preferably, the substrate is an insulating substrate, and the device further comprises means for suctioning the cell.

Preferably, the well for culturing the cell is made of a material selected from the group consisting of silicone, plastic, SiO₂, and rubber.

Preferably, the reference electrode is in the shape of a ring.

Preferably, the reference electrode is made of a conductive material.

Preferably, the insulating substrate comprises a throughhole having a diameter of 6 µm.

Preferably, the insulating substrate comprises a supporting layer, and the supporting layer is a SOI substrate having a thickness of at least 10 µm.

Preferably, the supporting layer is selected from the group consisting of silicon, plastic, SiO₂, and rubber.

Preferably, the supporting layer has a thickness of 1 µm or more.

Preferably, the cell suctioning means is made of silicone, plastic, SiO₂, and rubber, a thickness of the cell suctioning portion being 10 µm or more.

Preferably, an inner wall of the depression is treated to become hydrophilic.

In one aspect, the present invention relates to a method for measuring an extracellular potential. The method comprises the steps of providing a reaction system for measuring an electrical characteristic of a biological sample, disposing an intact cell of interest in the reaction system, and detecting an electrical characteristic of the cell of interest. The reaction system may be an extracellular potential measuring device comprising at least one well comprising means for holding a cell provided on a substrate, a measuring electrode for detecting an electrical signal of each of the at least one well, and a reference electrode.

Preferably, the electrical characteristic detecting step comprises detecting the electrical characteristic of the cell of interest at least twice, and comparing at least two values of the detected electrical characteristic.

Preferably, a capacity of a region, in which the measuring electrode is provided, is smaller than a capacity of a region, in which the reference electrode is provided.

Preferably, a surface area of the reference electrode is smaller than a surface area of the measuring electrode.

Preferably, an impedance of the measuring electrode is lower than an impedance of the reference electrode.

Preferably, the impedance of the measuring electrode at 10 Hz to 10 kHz is smaller than the impedance of the reference electrode at 10 Hz to 10 kHz.

Preferably, the substrate is an insulating substrate; the cell holding means is a throughhole immersed in an electrolyte and disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an amount of electrolyte immersing the reference electrode is different from an amount of electrolyte immersing the measuring electrode.

Preferably, the amount of electrolyte immersing the reference electrode is 5 times or more as great as the amount of electrolyte immersing the measuring electrode.

Preferably, the substrate is an insulating substrate; the cell holding means is a throughhole disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an electrode area of the reference electrode is different from an electrode area of the measuring electrode.

Preferably, the electrode area of the reference electrode is 1/5 or less of the electrode area of the measuring electrode.

Preferably, the substrate is an insulating substrate; the cell holding means is a throughhole disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an impedance of the reference electrode is different from an impedance of the measuring electrode.

Preferably, the impedance of the reference electrode is 5 times or more as great as the impedance of the measuring electrode.

Preferably, the distance of the reference electrode from the cell of interest is longer than the distance of the measuring electrode from the cell of interest.

Preferably, in the extracellular potential measuring device, the area of the reference electrode : the area of the measuring electrode = 1:5, the impedance of the reference electrode : the impedance of the measuring electrode = 5:1, or the volume of the electrolyte immersing the reference electrode : the volume of the electrolyte immersing the measuring electrode = 5:1.

Preferably, the extracellular potential is a signal associated with an activation of an ion channel or receptor of a cell, or an action of an intracellular signal transduction system.

Preferably, the electrical characteristic detecting step is performed in the presence of a standard chemical substance having a known action on the cell of interest and in the presence of a subject chemical substance, and the method further comprises the step of comparing electrical characteristics obtained in the presence of the standard chemical substance and in the presence of the subject chemical substance, and characterizing an action of the subject chemical substance on the biological sample.

Preferably, the cell of interest disposing step comprises the steps of introducing measuring solution into the well, generating a difference in pressure between the well and an opening of a throughhole opposite to the well, the throughhole being in communication with the well, and allowing the pressure difference to be a magnitude of 1/10 or less.

Preferably, the pressure difference is generated by reducing the pressure through the opening.

Preferably, the pressure difference is generated by applying pressure to the well.

Preferably, the pressure difference is generated by reducing the pressure through the opening and applying pressure to the well.

Preferably, the pressure difference is 0.01 to 0.5 atm.

In one aspect, the present invention relates to a quick drug screening apparatus, comprising the above-described extracellular potential measuring device, an electrical signal detecting section linked to an measuring electrode of the extracellular potential measuring device, and means for processing a signal, wherein the signal processing means processes a plurality of electrical signals from the electrical signal detecting section and displays an activity state of a cell.

Preferably, the quick drug screening apparatus may further comprise means for injecting or draining the subject drug solution, and means for transferring the extracellular potential measuring device.

Preferably, the step of detecting the electrical characteristic of the biological sample of interest comprises the steps of (a) recording a physiochemical signal emitted from the biological sample of interest as a time-series signal value, (b) sampling the time-series signal value to obtain a plurality of groups of extracted data consisting of a plurality of values, and calculating a standard deviation of each of the plurality of groups, (c) calculating an average of each of the standard deviations, and (d) detecting a change in the electrical characteristic of the cell of interest based on the average of the standard deviations.

Preferably, the step of detecting the electrical characteristic of the cell of interest comprises the steps of (a) recording an electrical signal emitted from the cell of interest as a time-series signal value, (b) sampling the time-series signal value to obtain a plurality of groups of extracted data consisting of a plurality of values, and calculating a standard deviation of each of the plurality of groups, (c) dividing the standard deviations into a plurality of classes having a predetermined size of standard deviation as a unit, and obtaining a distribution indicating electrical characteristics of the plurality of groups of extracted data belonging to the classes, (d) approximating the distribution to a normal distribution, (e) calculating an average and a half width of the resultant normal distribution, (f) optionally repeating (b) to (e), and (g) detecting a change in the electrical characteristic of the cell of interest based on the average and half width.

Preferably, the steps (b) to (e) are repeated and the number of the time-series signal values to be sampled is changed in each repetition.

Preferably, before the step (b), the method further comprises adding up the time-series signal values emitted by a plurality of cells of interest provided in the reaction systems.

Preferably, before the step (a), the method further comprises simultaneously stimulating the plurality of the cells of interest.

Preferably, the step of detecting a change in the electrical characteristic of the cell of interest comprises (a) recording an electrical signal emitted from the cell of interest as a time-series signal value, (b) sampling the time-series signal value to obtain a plurality of groups of extracted data consisting of a plurality of values, and calculating a standard deviation of each of the plurality of groups, (c) dividing the standard deviations into a plurality of classes having a predetermined size of standard deviation as a unit, and obtaining a distribution indicating electrical characteristics of the plurality of groups of extracted data belonging to the classes, (d) approximating the distribution by curvilinear approximating analysis selected from the group consisting of exponential decreasing analysis, exponential increasing analysis, Gaussian distribution, Lorentz distribution, σ analysis, multiple peak analysis, and nonlinear analysis, and (e) detecting a change in the electrical characteristic of the biological sample based on gradients before and after a peak on the approximated curve obtained by the step (d).

The sampling in the step (b) may be carried out in a time-series manner or at random.

Preferably, the sampling in the step (b) is carried out a plurality of times from initial data a in a time-series manner and a plurality of times from data b recorded at a predetermined time after the initial data a in a time-series manner.

Preferably, the sampling in the step (b) is carried out a plurality of times from initial data a in a time-series manner and a plurality of times from data b recorded at a predetermined time after the initial data a in a time-series manner.

Preferably, the step of detecting a change in the electrical characteristic of the cell of interest, comprises (a) recording an electrical signal emitted from the cell of interest as a time-series signal value, (b) sampling the time-series signal value to obtain a plurality of groups of extracted data consisting of a plurality of values, and calculating a standard deviation of each of the plurality of groups, (c) sampling the resultant standard deviations to obtain a plurality of groups of extracted standard deviations consisting of a plurality of values, and calculating an average of each of the plurality of groups of extracted standard deviations, and (e) obtaining an index value detecting a change in the electrical characteristic of the cell of interest based on a time of occurrence of the time-series signal value when the average reaches a predetermined threshold.

The electrical signal may be a signal associated with activation of an ion channel or receptor of the cell, or action of an intracellular signal transduction system.

In one aspect, the present invention relates to an apparatus for measuring an action of a subject chemical substance on a cell of interest, comprising a reaction system for measuring an electrical characteristic of the cell of interest, means for recording an electrical signal emitted from the cell of interest as a time-series signal value, means for sampling the time-series signal value to obtain a plurality of groups of extracted data consisting of a plurality of values, and calculating a standard deviation of each of the plurality of groups, means for calculating an average of each of the standard deviations, and means for detecting a change in the electrical characteristic of the cell of interest based on the average of each of the standard deviations.

In one aspect, the present invention relates to an apparatus for measuring an action of a subject chemical substance on a cell of interest, comprises a reaction system for measuring an electrical characteristic of the cell of interest, means for recording an electrical signal emitted from the cell of interest as a time-series signal value, means for sampling the time-series signal value to obtain a plurality of groups of extracted data consisting of a plurality of values, and calculating a standard deviation of each of the plurality of groups, means for dividing the standard deviations into a plurality of classes having a predetermined size of standard deviation as a unit, and obtaining a distribution indicating electrical characteristics of the plurality of groups of extracted data belonging to the classes, means for approximating the distribution to a normal distribution, means for calculating an average and a half width of the resultant normal distribution, and means for detecting a change in the electrical characteristic of the cell of interest based on the average and half width.

In one aspect, the present invention relates to an apparatus for measuring an action of a subject chemical substance on a cell of interest, comprising a reaction system for measuring an electrical characteristic of the cell of interest, means for recording an electrical signal emitted from the cell of interest as a time-series signal value, means for sampling the time-series signal value to obtain a plurality of groups of extracted data consisting of a plurality of values, and calculating a standard deviation of each of the plurality of groups, means for dividing the standard deviations into a plurality of classes having a predetermined size of standard deviation as a unit, and obtaining a distribution indicating electrical characteristics of the plurality of groups of extracted data belonging to the classes, means for approximating the distribution by curvilinear approximating analysis selected from the group consisting of exponential decreasing analysis, exponential increasing analysis, Gaussian distribution, Lorentz distribution, σ analysis, multiple peak analysis, and nonlinear analysis, and means for detecting a change in the electrical characteristic of the cell of interest based on gradients before and after a peak on the obtained approximated curve.

In one aspect, the present invention relates to an apparatus for measuring an action of a subject chemical substance on a cell of interest, comprising a reaction system for measuring an electrical characteristic of the cell of interest, means for recording an electrical signal emitted from the cell of interest as a time-series signal value, means for sampling the time-series signal value to obtain a plurality of groups of extracted data consisting of a plurality of values, and calculating a standard deviation of each of the plurality of groups, means for sampling the resultant standard deviations to obtain a plurality of groups of extracted standard deviations consisting of a plurality of values, and calculating an average of each of the plurality of groups of extracted standard deviations, and means for obtaining an index value detecting a change in the electrical characteristic of the cell of interest based on a time of occurrence of the time-series signal value when the average reaches a predetermined threshold.

### BRIEF DESCRIPTION OF THE DRAWINGS

Figure **1** is a diagram showing a structure of a well of an extracellular potential measuring device according to the present invention, comparing with a well of a device using a conventional microelectrode. **(A)** shows the structure of the well of the extracellular potential measuring device of the present invention. **(B)** shows the well of the device using the conventional micro planar electrode.

Figure **2** is a diagram showing an exemplary method for producing an extracellular potential measuring device according to the present invention.

Figure **3** is an electron micrograph showing a depression and a throughhole provided on a substrate of the extracellular potential measuring device of the present invention.

Figure **4** is a diagram roughly showing the extracellular potential measuring device of the present invention.

Figure **5** is a diagram showing a variant example of the extracellular potential measuring device of the present invention.

Figure **6** is a diagram roughly showing a quick drug screening apparatus according to the present invention.

Figure **7** is a diagram roughly showing a detecting section of the quick drug screening apparatus of the present invention.

Figure **8** is a diagram showing a result of a test conducted using the extracellular potential measuring device of the present invention.

Figure **9** is a diagram showing a result of a test conducted using the extracellular potential measuring device of the present invention.

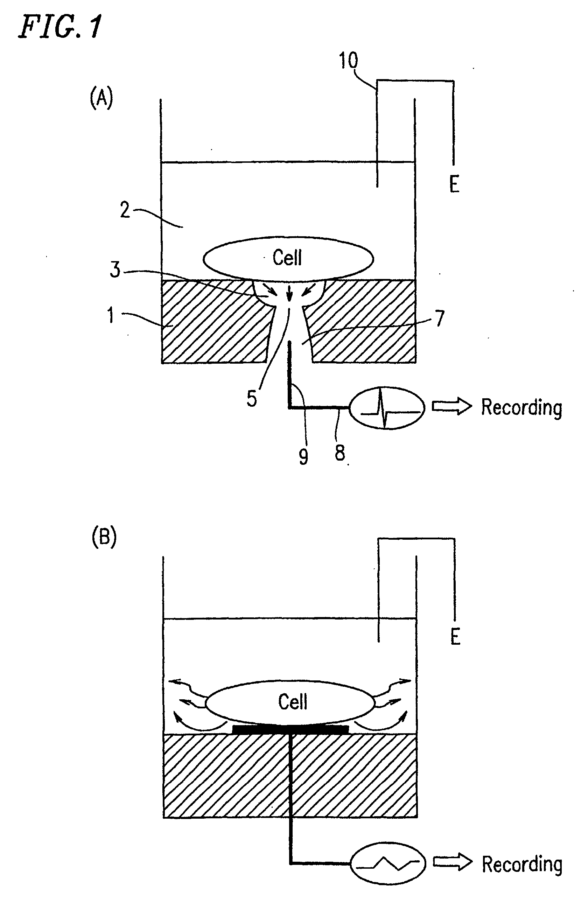

Note that reference numerals shown in Figures **1** to **9** indicate the following members, respectively.

**1** substrate; **2** well; **3** depression; **5** throughhole opening; **7** throughhole; **8** wire; **9** measuring electrode; **10**, **13, 33** SiO₂ layer; **11, 12** Si layer; **15** Al layer; **20** ring-shaped reference electrode; **22** well side wall; **24** wire; **29** measuring electrode; **31,32** protection film.

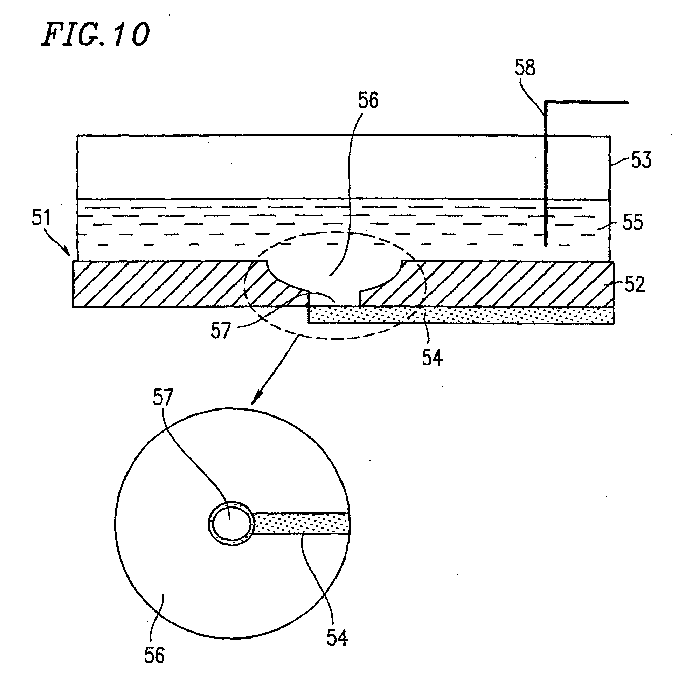

Figure **10** is a diagram roughly showing a variant example of the extracellular potential measuring device of the present invention.

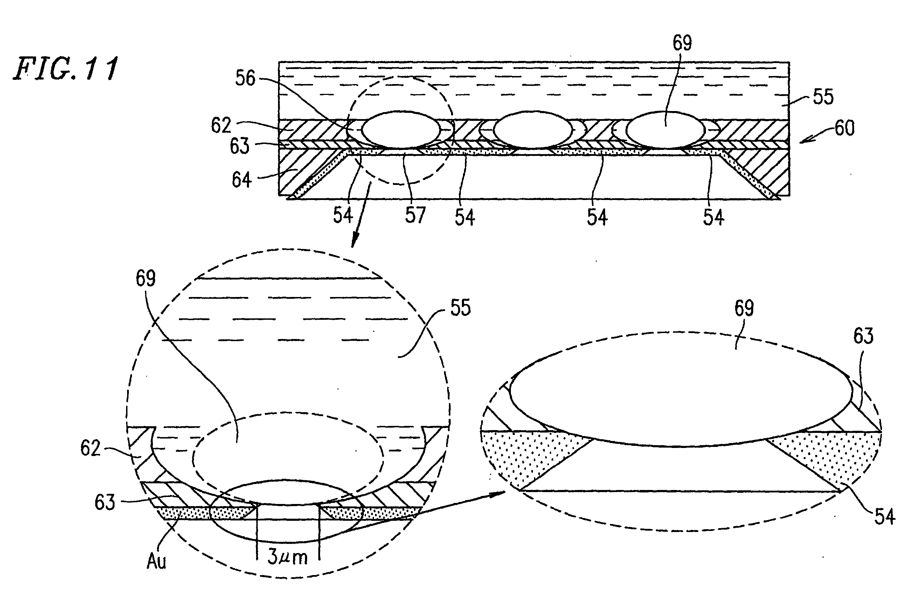

Figure **11** is a diagram roughly showing another variant example of the extracellular potential measuring device of the present invention.

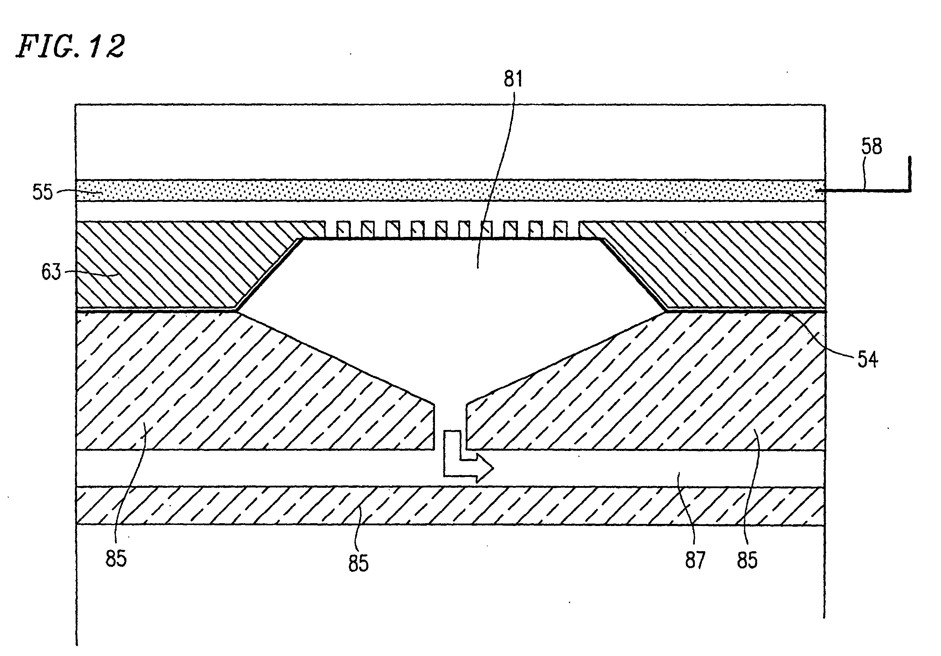

Figure **12** is a diagram roughly showing another variant example of the extracellular potential measuring device of the present invention.

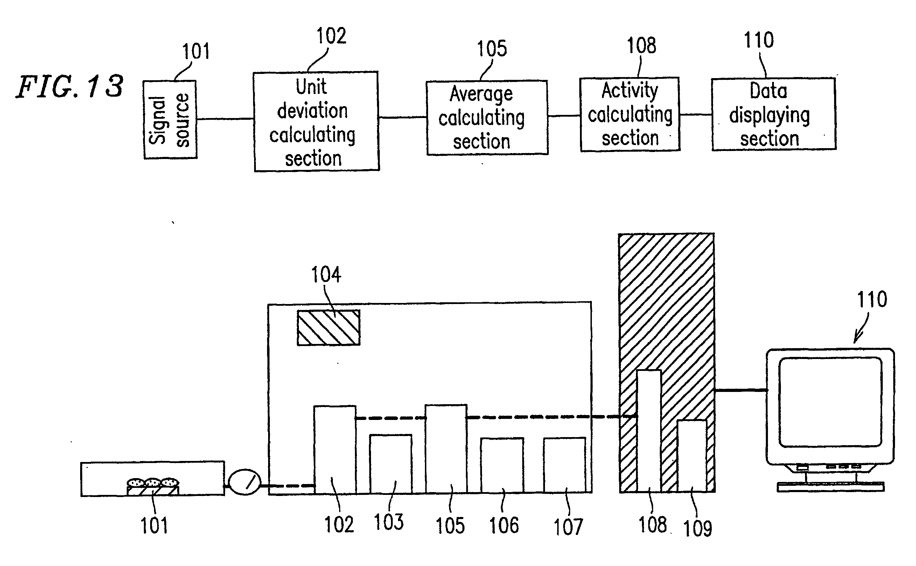

Figure **13** is a diagram roughly showing an apparatus according to the present invention.

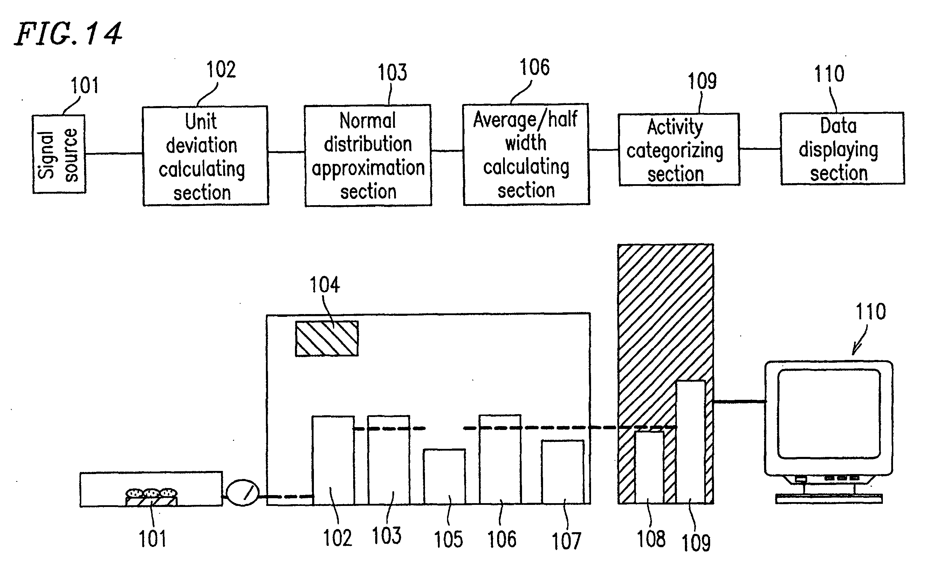

Figure **14** is a diagram roughly showing another apparatus according to the present invention.

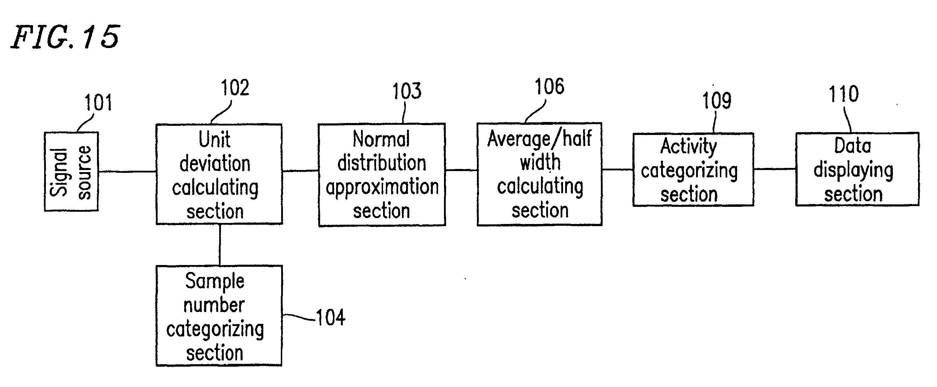

Figure **15** is a diagram roughly showing another apparatus according to the present invention.

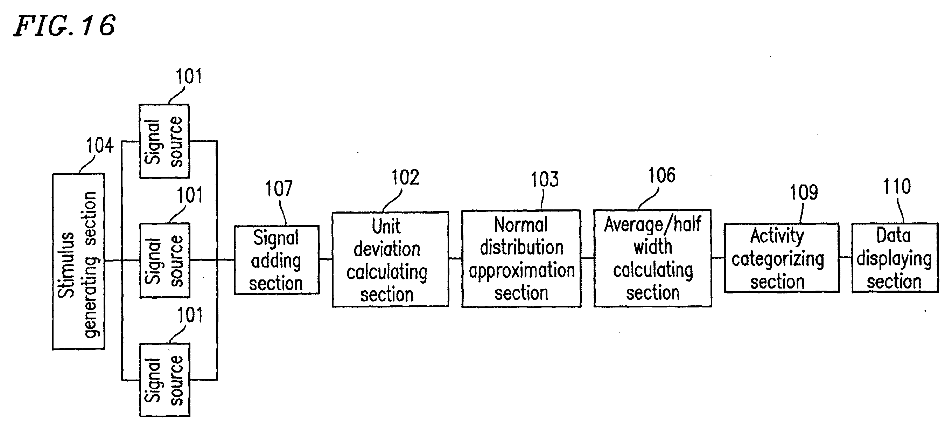

Figure **16** is a diagram roughly showing another apparatus according to the present invention.

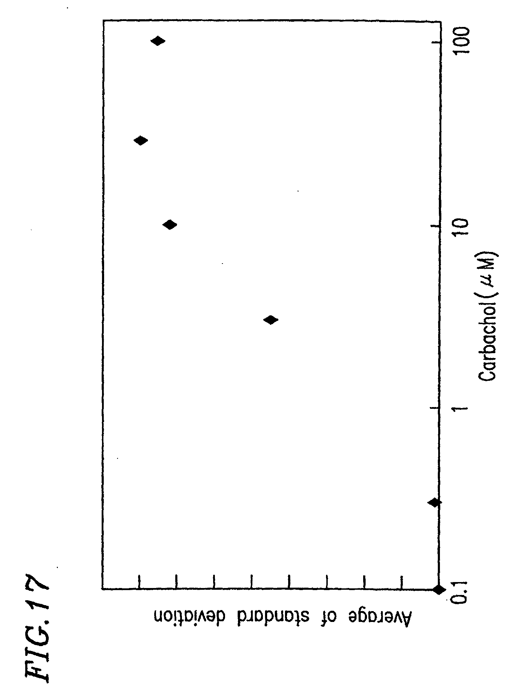

Figure **17** is a diagram showing a concentration-dependent reaction of a Lymnaea Stagnalis neuron to Carbachol, which was measured by the apparatus of the present invention.

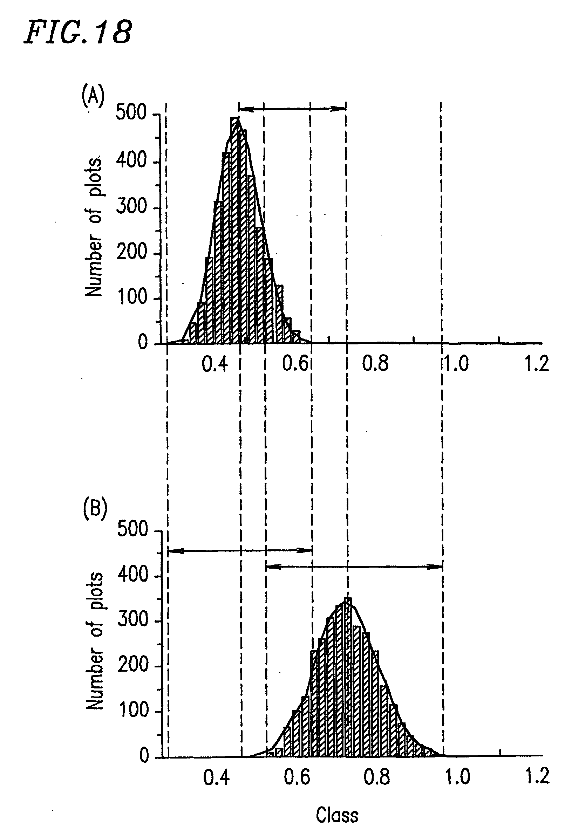

Figure **18** is a diagram showing a result of measuring a Lymnaea Stagnalis neuron before and after applying Carbachol thereto, which was measured by the apparatus of the present invention.

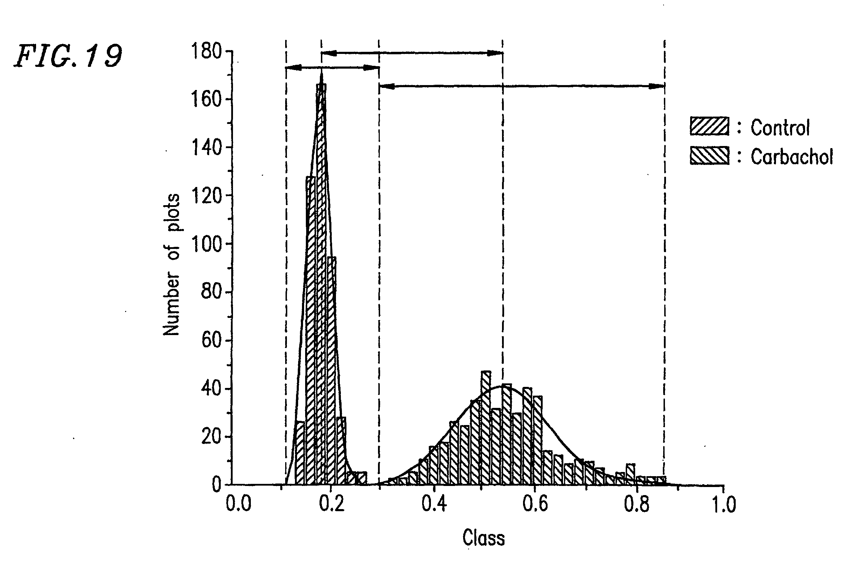

Figure **19** is a diagram showing a result of measuring a Lymnaea Stagnalis neuron before and after applying Carbachol thereto, which was measured by a conventional intracellular recording method.

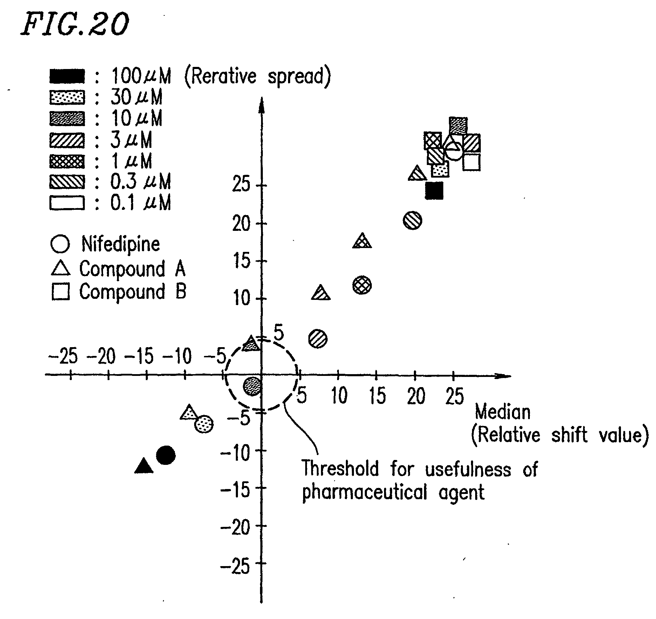

Figure **20** is a diagram showing a method for categorizing an effect of a drug according to the present invention.

Note that reference numerals shown in Figures **10** to **20** indicate the following members, respectively.

**51, 60** substrate; **52** SOI substrate; **53** well; **54** measuring electrode; **55** culture medium; **56** depression; **57** throughhole; **58** reference electrode; **62** Si layer; **63** SiO₂ layer; **64** supporting substrate; **69** cell; **81** space in communication with suctioning line; **85** suctioning line attachment; **87** cell suctioning line; **101** signal source; **102** unit deviation calculating section; **103** normal distribution approximation section; **104** trigger signal generating section; **105** average calculating section; **106** average/half width calculating section; **107** signal adding section; **108** activity calculating section; **109** activity categorizing section; **110** data displaying section; **111** sample categorizing section.

### BEST MODE FOR CARRYING OUT THE INVENTION

The present inventors used a micromachining technique to form a plurality of minute depressions on the bottom side of each well of a multi-titer plate (96, 384, 1536 wells), which is generally employed for a quick drug screening method. The present inventors found that by holding a cell in the depression, data having quality close to that obtained by conventional patch clamp methods can be obtained. The present invention was thus achieved.

The present invention solves the above-described problems in the prior art. The present invention provides an extracellular potential measurement device comprising a sensor substrate capable of simply and highly reliably detecting a minute electrical signal emitted by a biological sample, which cannot be detected by conventional extracellular recording methods, and a method and apparatus for measuring an electrochemical change in a biological sample using the device. An object of the present invention is to provide a method for measuring the macro channel activity of an entire cell, i.e., a physicochemical change generated by a cell by using a simple sensor substrate not particularly requiring a dedicated control apparatus, whereby the cell can be easily measured in a short time simply by disposing the cell on a well in a chamber without forming a gigaseal on an insulating substrate, and since no chemical is used, it is not necessary to consider side effects or changes in fluorescence sensitivity over time; and a quick drug screening method using that method.

Figure **1(A)** schematically shows a structure of a well of an extracellular potential measuring device according to the present invention. A well **2** contains culture medium. A subject cell represented by an ellipse at the middle of Figure **1(A)** is captured or held by a cell holding means provided on a substrate **1**. The cell holding means comprises a depression **3** formed on the substrate **1**, and a throughhole **7** which is in communication with the depression **3** through an opening **5**.

In Figure **1(A)**, a measuring electrode **9**, which is a sensor means, is disposed on a side of the throughhole **7** opposite to the cell. The measuring electrode **9** is linked via a wire **8** to a signal detecting section. The throughhole **7** constitutes a part of a suctioning portion which is in communication with a suctioning means, such as a suctioning pump, and is linked to a cell suctioning means (not shown). The cell suctioning means hermetically holds and suctions the cell membrane of the cell held by the depression **3** and the substrate **1** so as to prevent an electrical signal obtained through the cell membrane in the depression **3** from leaking into the culture medium in the well, as indicated by arrows in Figure **1(A)**.

In Figure **1(A)**, a part of the cell is fitted into the opening of the depression **3**. The tightness between the cell membrane and the substrate **1** forms an electrical seal effective for the measurement of the extracellular potential, improving the quality of a detected electrical signal.

In the present invention, an assay system comprises a first region in which a reference electrode is disposed, a second region which is an intact cell, and a third region in which the measuring electrode is disposed. The assay system is used to measure the cell membrane potential of the intact cell or a change in the cell membrane potential occurring in the intact cell as a change in voltage due to a change in the extracellular potential.

For comparison, Figure **1(B)** schematically shows a case when a conventional planar microelectrode is used to measure an extracellular potential. In this case, most electrical signals emitted from a cell leak into culture medium, resulting in a significant reduction in the signal detection sensitivity.

The well shown in Figure **1(A)** is provided with a reference electrode **10**. The measuring electrode **9** is used to measure a cellular electrical signal with reference to a reference potential of the reference electrode **10**. Typically, the reference electrode **10** is a wire whose section has a diameter of about 100 µm to about 1000 µm and which is made of a material, such as gold, platinum, and silver-silver chloride. The reference electrode **10** may be in any size and shape, as required. Further, one or more reference electrodes **10** may be provided per well, as required, whereby the measurement precision of the extracellular potential may be improved.

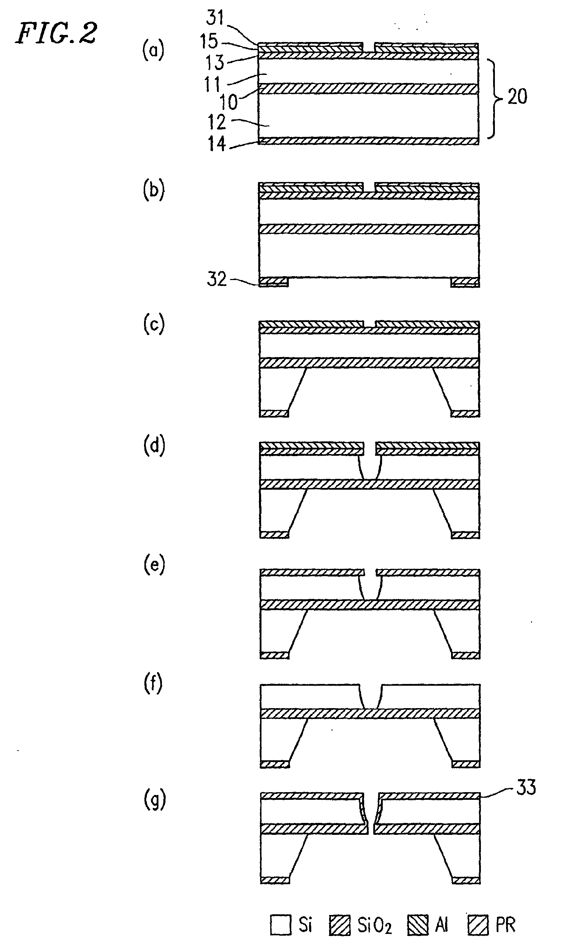

A device for measuring cellular activity comprising one or more wells as described above is produced using a conventional micromachining technique. Figure **2** shows an exemplary production process.

Initially, opposite sides of a SOI layer **20** comprising two Si layers **11** and **12** sandwiching a SiO₂ layer **10** are subjected to thermal oxidation to form SiO₂ layers **13** and **14**. Thereafter, on a surface of the SiO₂ layer **13**, an Al layer **15** is formed by deposition and is thereafter covered with a protection film **31** (photoresist: PR). Thereafter, the protection film **31** is subjected to patterning by photolithography, and the protection film **31** is then used as a mask to subject the Al layer **15** to patterning (Figure **2(a)**).

Next, the SiO₂ layer **14** formed on the rear surface of the substrate is covered with a protection film **32** and thereafter the protection film **32** is subjected to patterning by photolithography, and the protection film **32** is used as a mask to remove the SiO₂ layer **14** while leaving a part thereof (Figure **2(b)**). After the removal of the protection films **31** and **32**, the SiO₂ layer **14** is used as a mask to subject the Si layer **12** on the rear surface of the substrate to wet etching using tetramethylammonium hydroxide (TMAH) (Figure **2(c)**). Further, the SiO₂ layer **13** and the Si layer **11** on the front surface of the substrate are subjected to dry etching using reactive ion etching (RIE) where the Al layer **15** is used as a mask (Figure **2(d)**). Next, after the removal of the Al layer **15** from the front surface of the substrate (Figure **2(e)**), the SiO₂ layer **10** in the middle of the substrate **20** is etched by dry etching using RIE from the front surface side where the Si layer **11** is used as a mask, so that a hole is formed from the front surface through the rear surface of the substrate (Figure **2(f)**). The entire substrate **20** is subjected to thermal oxidation so that a SiO₂ layer **33** is deposited on a surface and an outermost surface of the device (Figure **2(g)**). Thereafter, an electrode is patterned on the rear surface SiO₂ layer **14** using vacuum deposition or sputtering. Examples of the preferable material include gold, platinum, platinum black, palladium, and silver. Alternatively, in order to deposit the above-described material on the outermost surface, another metal layer may be deposited between the material and the SiO₂ layer **14**. A material preferably used for this purpose includes nickel, chromium, ITO, tungsten, titanium, tin, manganese, lead and an alloy thereof. Alternatively, on the substrate **20**, after an electrode is patterned, an insulating layer may be formed on the electrode layer other than the electrode portion. Examples of the preferable material include polyimide resin, PMMA (polymethylmethacrylate), acrylic resin, PDMS (polydimethylsiloxane), epoxy resin, materials causing these materials to be photosensitive, and photosensitive photoresist.

Note that although in Figure **2**, a single depression is produced on a substrate, one or more depressions can be produced by selecting a desired pattern in steps (a) and (b) in Figure **2**.

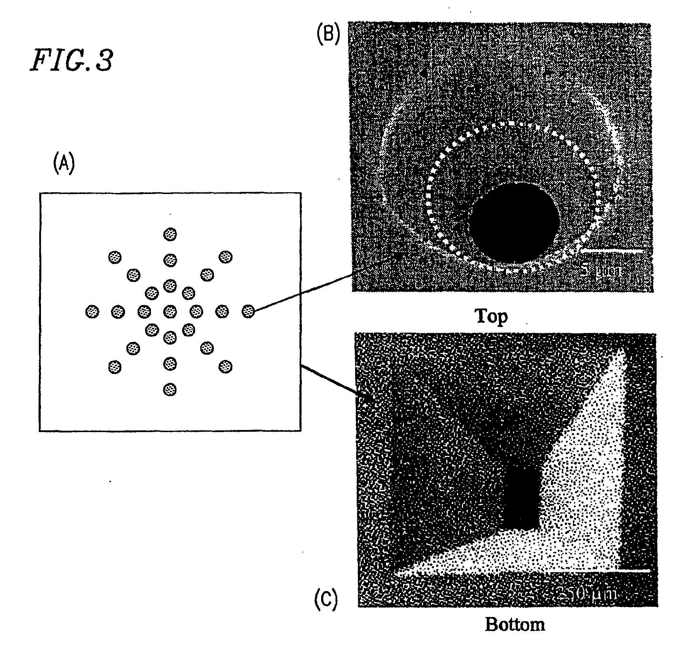

Figure **3(A)** shows a case when a total of 25 depressions per well (one set) are produced and arranged in the shape of an asterisk.

Figure **3(B)** is an electron micrograph of a single depression thus produced. A scale bar at a lower right of the picture indicates 5 µm. In this example, the opening of a depression **3** is substantially in the shape of a circle having a diameter of about 20 µm, and a hole having a diameter of about 7 µm is observed substantially at the middle of the bottom of the depression. Note that a dotted line represents a position at which a cell is disposed. Figure **3(C)** is an electron micrograph taken from the rear side of the substrate. A scale bar at a lower right of the picture indicates 250 µm. In this example, a rectangle of about 100 µm × about 150 µm formed by etching is observed at the center of the picture. In this region, 25 throughholes as described above are provided.

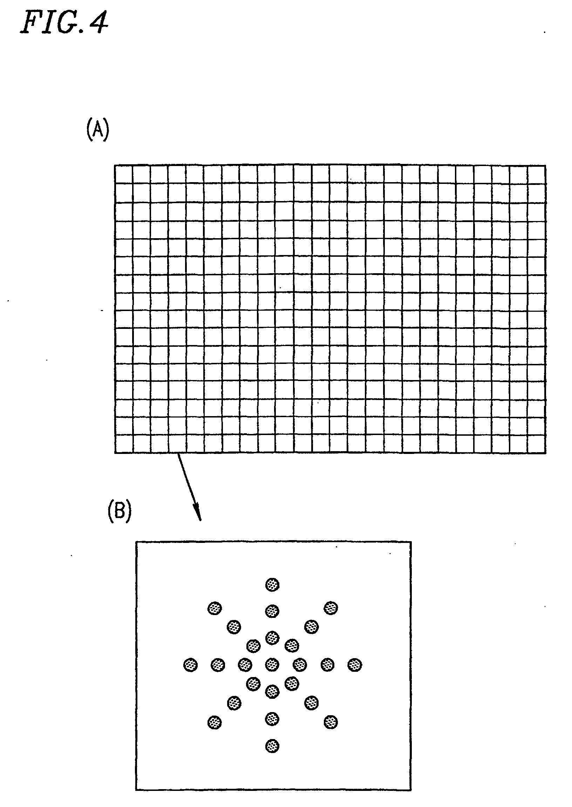

The above-described set of depressions on the substrate are integrally attached or adhered to a plate having a plurality of holes which is produced compatibly to the shape of a multi-titer plate (96, 384, 1536 well plates and the like) which is generally used for a quick drug screening method (shown in Figure **4(A)**, hereinafter referred to as a plate). In this case, one set of depressions are formed on the substrate in such a manner as to be accommodated in each of the plurality of holes on the plate (Figure **4(B)**). In the example shown in Figure **4**, 24 × 16 (=384) sets of depressions are formed in such a manner as to match the plate. The hole of the plate has substantially the same size as that of a well of a generally used microtiter plate, so that one set of depressions (Figure **4(B)**) are formed in a region of about 4 mm². Typically, individual depressions are spaced by 10 to 100 µm.

The above-described plate is tightly placed or adhered onto the resultant substrate so that an extracellular potential measuring device having a plurality of wells is obtained. The well is composed of a side wall, which is of a hole of the plate, and a bottom side, which is of the substrate. The adhesion of the plate and the substrate is caused by one-component RTV rubber (ShinEtsu Silicones KE42T) or the like.

Further, a plate having a plurality of holes, which is produced to fit the shape of the multititer plate, may be made of a low cytotoxic material, such as Teflon, polystyrene, polycarbonate, and polyethylene terephthalate, and may be formed by a method, such as extrusion, well known to those skilled in the art.

Alternatively, when a silicon wafer, which is easy to micromachine, is used as a material for the substrate, it is possible to form a well shape as shown in Figure **1(A)** directly on the silicon wafer substrate without a plate mimicking a multititer plate.

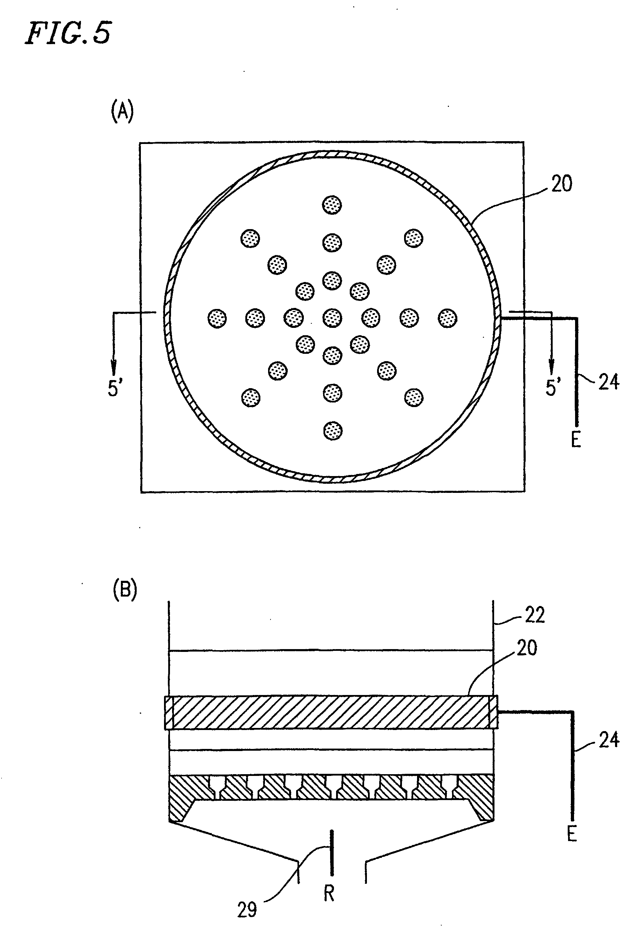

Figure **5** shows an extracellular potential measuring device comprising a ring-shaped reference electrode **20** at a side wall **22** of a well, as a variant example of the present invention. Figure **5(A)** is a plan view of the device. Figure **5(B)** is a cross-sectional view of the device, taken along line **5**' shown in Figure **5(A)**. In this variant example, since the reference electrode is in the shape of a ring, a reference potential is provided in a well uniformly, thereby improving measurement precision. The ring-shaped electrode **20** is formed of, for example, a wire having a line width of about 1 µm to about 1000 µm and made of gold, platinum, silver-silver chloride or the like. Reference numeral **24** indicates a wire linking the reference electrode to a power source. A measuring electrode **29** is linked via a wire (not shown) to a signal detecting section, as in the device shown in Figure **1**.

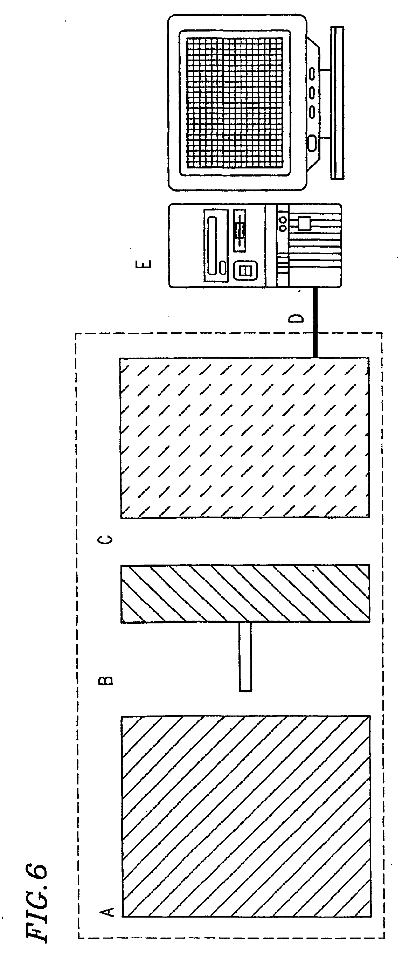

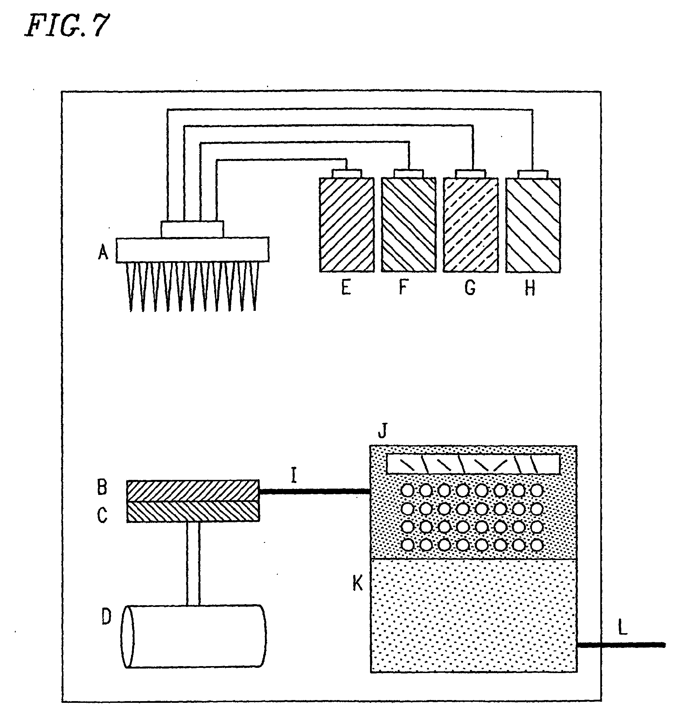

Figure **6** schematically shows an exemplary configuration of a quick drug screening apparatus incorporating the extracellular potential measuring device of the present invention therein. The configuration of Figure **6** is described below. **A** indicates a culture apparatus which accommodates the extracellular potential measuring device and maintains environment suitable cell culture depending on the subject. **B** indicates a means for transferring the extracellular potential measuring device from the culture apparatus to a signal detecting apparatus. For example, a robot arm may be used as the transferring means. **C** indicates the signal detecting apparatus whose configuration is shown in detail in Figure **7**. **D** indicates a signal drawing cable. **E** indicates a signal processing apparatus (computer).

Figure **7** schematically shows an exemplary configuration of the signal detecting apparatus of Figure **6**. The configuration of Figure **7** is described below. **A** indicates a means for injecting or draining a subject drug solution (e.g., reagent dispensing multipipette). **B** indicates the above-described extracellular potential measuring device. **C** indicates a suctioning section. **D** indicates a suctioning pump. **E** to **H** indicate reagents. **I** indicates a signal drawing line. **J** indicates a preamplifier. **K** indicates a main amplifier. **L** indicates a signal drawing line connected to a signal processing means (computer).

Note that the above-described components are linked by a known means in the art, and the linkage is not particularly specified.

Next, a variant example of the present invention will be described with reference to Figures **10** and **11**. Figure **10** is a diagram roughly showing a variant example of the extracellular potential measuring device of the present invention. A sensor substrate **51** is provided with a well **53**, in which a cell is placed, on a top surface thereof via a SOI substrate **52**, and a measuring electrode **54** for detecting a signal, which is made of representatively gold, on a bottom surface thereof. About 50 µl of culture medium **55** is present in the well **53**. About 1 µl or less of culture medium is present in a throughhole **57** on the SOI substrate **52**. A depression **56** is formed in the well **53** to have an optimum structure for holding a cell. where the diameter of an opening is representatively about 10 µm. In the well **53**, a reference electrode **58** (representatively, Ag-AgCl) is immersed in the culture medium **55**.

As used herein, the well **53** accommodating the reference electrode **58**, the culture medium **55** immersing the reference electrode **58**, and the depression **56** are collectively referred to as a reference electrode arrangement environment. The culture medium in the vicinity of the measuring electrode **54** and the culture medium contained in the throughhole **57** are collectively referred to as a measuring electrode arrangement environment.

The size of the depression **56** may be changed depending on the biological sample of interest, and is not particularly limited as long as the biological sample of interest is maintained. Representatively, the opening of the depression **56** has a diameter of 10 to 500 µm and a depth of 1 to 500 µm. Typically, the opening of the depression **56** has a diameter of 10 to 100 µm and a depth of 2 to 100 µm. As a preferable illustrative depression, for example, a depression having an opening having a diameter of 20 µm and a depth of 10 µm, and another depression having an opening having a diameter of 20 µm and a depth of 20 µm are exemplified Further, the size of the throughhole **57** is not particularly limited as long as the biological sample of interest is not passed through the throughhole **57** and is maintained in cooperation with the depression **56**. Representatively, the opening of the throughhole **57** has a diameter of 5 to 100 µm, and a depth of 10 nm to 100 µm. As an illustrative throughhole, for example, a throughhole having a diameter of 5 µm and a depth of 1.5 µm is exemplified.

Figure **10** also shows an enlarged plan view of the depression **56** at a lower portion thereof.

Figure **11** is a diagram roughly showing another variant example of the extracellular potential measuring device of the present invention. A sensor substrate **60** shown in Figure **11** is different from the sensor substrate **51** shown in Figure **10** in that the sensor substrate **60** is provided with a plurality of depressions **56** and throughholes **57**. As shown in Figure **11**, an elliptical cell is held in each depression **56**. Insulating substrates **62** to **64** are made of SOI. The SiO₂ layer **63** is provided under the Si layer **62**, and the supporting substrate **64** is provided under the SiO₂ layer **63**. A measuring electrode **54** is provided on a bottom surface of the sensor substrate **60** and along part of a surface of the supporting substrate **64** and a surface of the SiO₂ layer **63**, where the measuring electrode **54** contacts cells **69**, or is located in the vicinity of the cells **69**, preferably a distance of 10 µm or less from the cells **69**.

In the extracellular potential measuring device of the present invention, only electrolyte suctioned by a cell suctioning means is present in the vicinity of the measuring electrode **54**. Therefore, in the extracellular potential measuring device of the present invention, electrolyte solution present in the vicinity of a side of the substrate **60** opposite to the other side on which the biological sample of interest is placed, i.e., the bottom surface of the sensor substrate **60** is not more than about 1 to 10 µl including electrolyte solution filling throughholes **57**. Further, it is not particularly necessary that the entire cell suctioning system is filled with electrolyte, as long as an electrical change in the cell can be detected by the measuring electrode **54**.

As shown in Figures **10** and **11**, one depression **56** and one throughhole **57** may be provided per measuring electrode, or a plurality of depressions **56** and throughholes **57** may be provided per measuring electrode.

Figure **12** is a diagram roughly showing a suctioning line attachment **85** which is optionally used when an electrical change in a cell is measured using the sensor substrate **60** shown in Figure **11**. As shown in Figure **12**, the suctioning line attachment **85** constitutes a part of a cell suctioning means provided on the bottom surface of the sensor substrate **60**. The suctioning line attachment **85** is made of a material, such as acrylic, PMDS, or silicone rubber, and is shaped to form a space **81**, which is communication with a suctioning line, corresponding to a plurality of throughholes **57** provided in the sensor substrate. The suctioning line attachment **85** is attached to the bottom surface of the sensor substrate by silicone rubber as amedium. Further, the suctioning line attachment **85** can be integrally adhered to the sensor substrate. As shown in Figure **12**, when measuring an electrical change in a cell, it is preferable that the cell is tightly attached to the insulating substrate with suctioning pressure by a cell suctioning system line **87**. Note that in Figure **12**, a cell subject is omitted and structures of the depression and the throughhole are shown in a simplified manner.

Figure **13** is a conceptual diagram showing a configuration of an apparatus for measuring an action of a subject chemical substance on a biological sample, which comprises the sensor substrate of the present invention. This apparatus comprises a measuring section (signal source) **101** comprising the sensor substrate of the present invention, a unit standard deviation calculating section **102** for sampling a signal from the measuring section **101** and calculating a standard deviation, an average calculating section **105** for calculating an average of the resultant standard deviations, an activity calculating section **108** for calculating an ion channel activity of a cell from an average standard deviation output from the average calculating section **105**, and a data displaying section **110** for displaying the resultant activity. Communication between each section is indicated by a dotted line or a solid line in Figure **4**. Note that, representatively, the unit standard deviation calculating section **102**, the average calculating section **105** and the activity calculating section **108** are programs for executing the calculations, which are stored in a hard disk incorporated into a computer. The data displaying section **110** is a CRT.

Note that in Figure **13**, reference numerals **103, 104, 106** and **109** indicate a normal distribution approximation section, a stimulus generating section an average/half width calculating section, and an activity categorizing section, which are described below.

### Examples

The present invention will be described by way of examples. The examples below illustrate the present invention but are not intended to limit the present invention.

### (Example 1)

25 depressions with a throughhole were formed on a silicon substrate, spaced by an interval of 100 µm (distance between each center of the hole) in the shape of an asterisk as shown in Figure **3**. Each depression had an opening having a diameter of 20 µm and a depth of 10 µm. A throughhole having a diameter of 3 µm was provided at a center portion of the depression. A plate (width × length × thickness = 12.7 × 8.1 × 7 mm) having a sufficient size to have holes which can contain the depressions was adhered onto the substrate. The resultant extracellular potential measuring device was incorporated into the apparatus shown in Figure **6**.

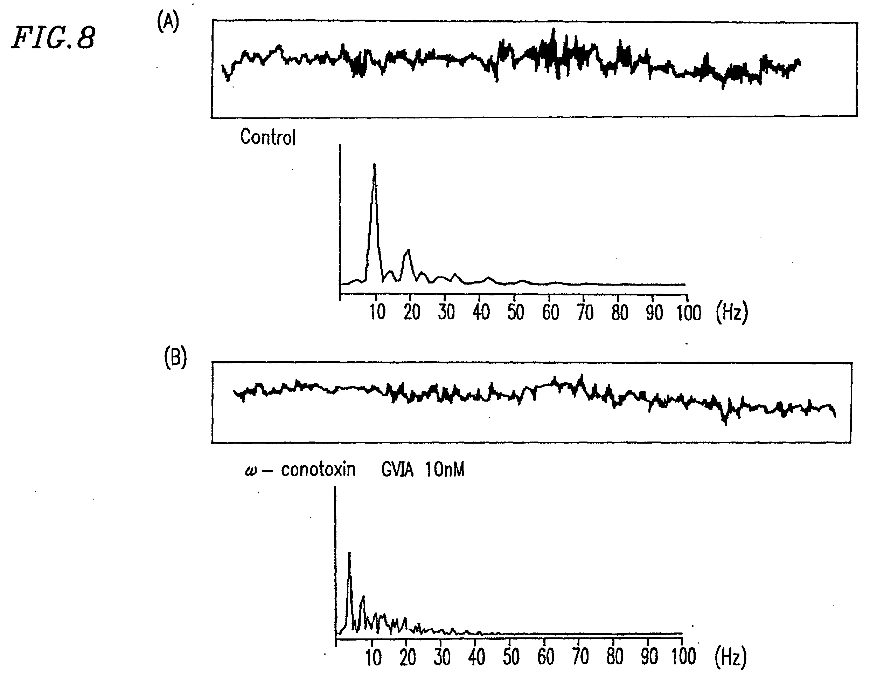

Neurons were prepared from the cerebral cortex of gestation day 17 SD rat embryos and were suspended in Dulbecco's modified medium to a concentration of 1 × 10⁵ cells/ml, and were dispensed into wells. The cells were trapped by each depression, and were suctioned slightly by a suctioning syringe so as to improve the tightness between the cells and the silicon substrate. In this situation, the cells were cultured for 30 minutes in a CO₂ incubator (34°C, 5% CO₂). Thereafter, a Ca antagonist (ω-conotoxin GVIA) was added to the wells to a concentration of 10 nM, and the cells were allowed to react for 5 minutes. Next, glutamic acid was added to 20 µM. The resultant occurring noise was compared with that which was generated in the absence of the Ca antagonist. The result is shown in Figure **8**.

Figure **8(A)** shows a chart showing the noise observed in control cells measured in the absence of the Ca antagonist (the horizontal axis represents time and the vertical axis represents voltage (the intensity of potential indicating the activity of a cell)), and a chart showing a frequency characteristic of the noise obtained by subjecting the noise to FFT (Fast Fourier Transform)(the horizontal axis represents the frequency of the noise and the vertical axis represents the square value of the amplitude of the noise (so-called power spectrum)). Figure **8(B)** is a chart showing the noise measured in the presence of the Ca antagonist, and a chart showing a frequency characteristic of the noise.

As shown in Figure **8**, the noise has a maximal value at about 4 Hz in the presence of the Ca antagonist, and at about 10 Hz in the absence of the Ca antagonist. For intensity, it was observed that the noise in the absence of the Ca antagonist was about 1.5 times as great as that in the presence of the Ca antagonist. The influence of the Ca antagonist on the cell could be detected significantly.

### (Example 2)

An extracellular potential measuring device having 96 wells, each of which having the same structure as that of Example 1, was produced. Specifically, a set of 25 depressions having an opening having a diameter of 20 µm were provided on a silicon wafer substrate in the shape of an asterisk. Sets of depressions were spaced at intervals of about 10 mm in a 8 × 12 matrix. A plate having 96 holds (width × length × thickness = 12.7 × 8.1 × 7 mm), which was made of polystyrene, was adhered onto the silicon wafer substrate so that each hole of the plate contained one set of depressions. The resultant 96-well extracellular potential measuring device was incorporated into the apparatus shown in Figure **6**. Note that each depression was provided with a throughhole having a diameter of 3 µm at the center thereof.

As in Example 1, neurons prepared from gestation day 17 rat embryos prepared were dispensed into each well to a concentration of 1 × 10⁵ cells/ml. Further, applying pressure and suctioning were repeated in a stepwise manner at a top portion of the well and at a lower side of the throughhole in the device, thereby improving the tightness between the cell and the silicon wafer substrate. In this situation, the cells were cultured for 30 minutes in a CO₂ incubator. Thereafter, two Ca antagonists (ω-conotoxin and ω-agatoxin) were added to the cells to final concentrations of 1, 3, 10 and 30 nM, and were allowed to react for 5 minutes. Glutamic acid was added to 20 µM. The resultant occurring noise was compared with that which was generated in the absence of the Ca antagonist. Signal processing software was used to detect the presence or absence of an effect of the Ca antagonist on the cells.

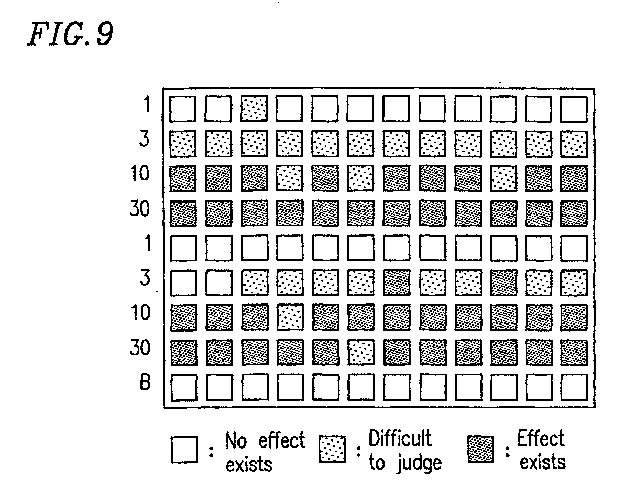

Figure **9** shows the result displayed on a computer screen. The matrix-like screen display has a one-to-one correspondence with the well matrix of the extracellular potential measuring device. Numbers to the left of Figure **9** represent the concentration of the Ca antagonist which was added to the well on each row of the matrix. Note that B indicates a row of control wells to which the Ca antagonist was not added. In Figure **9**, the wells were displayed in white or black density. Detected signals were processed by signal processing software to display the difference in noise between the wells with respect to the noise signal observed in the control wells in different densities.

As shown in Figure **9**, it was found that with the extracellular potential measuring device of the present invention, a reaction dependent on the concentration of a Ca antagonist was obtained in each well, thereby detecting an effect of the Ca antagonist in multiple wells.

### (Example 3)

The measurement section (signal source) **101** comprises the sensor substrate shown in Figure **12**. The apparatus having the configuration shown in Figure **13** was used to measure an action of a neuron to the chemical substance Carbachol, where the neuron was prepared from Lymnaea Stagnalis. Carbachol is a chemical substance known as an analog of the neurotransmitter Acetylcholine. Carbachol (manufactured by Sigma) was dissolved in artificial cerebrospinal fluid to concentrations of 0, 0.1, 0.3, 1, 3, 10, 30 and 100 µM. The solutions having these concentrations were used to measure an electrical signal emitted by the neuron. For each Carbachol concentration, time-series data for 10 seconds was obtained from the measurement section (signal source) **101** comprising the sensor substrate, and was sampled at intervals of 100 miliseconds, and the standard deviation of the sampling data was calculated. The average of the standard deviation was plotted in Figure **17**.

As can be seen from Figure **17**, the greater the Carbachol concentration, the greater the average of the standard deviation every 100 miliseconds. This result indicates that the ion channels of a Lymnaea Stagnalis neuron were activated depending on the Carbachol concentration.

### (Example 4)

Figure **14** is a conceptual diagram showing a configuration of an apparatus for detecting an action of a subject chemical substance on a biological sample according to the present invention. The apparatus is the same as the apparatus of Figure **13**, except that a normal distribution approximation section **103**, an average/half-width calculation section **106** and an activity categorization section **109** are employed in place of the average calculation section **105** and the activity calculation section **108**. Communications between each section are indicated by a dashed line or a solid line in Figure **13**.

The normal distribution approximation section **103** divides a plurality of standard deviation values obtained by the unit standard deviation calculation section **102** into a plurality of classes having a predetermined width of standard deviation as a unit, plots the standard deviation values where the X axis represents the class and the Y axis represents the number of standard deviation values belonging to the class, and approximates the obtained graph to a normal distribution. The average/half-width calculation section **106** calculates the average and half-width of the resultant normal distribution. The activity categorization section **109** categorizes an ion channel activity based on the obtained average and half-width. Note that similar to the apparatus of Figure **13**, the unit standard deviation calculation section **102**, the normal distribution approximation section **103**, the average/half-width calculation section **106** and the activity categorization section **109** may be representatively software programs which are recorded in a hard disk of a computer. The data displaying section **110** may be a CRT.

The measurement section (signal source) **101** comprises the sensor substrate shown in Figure **12**. The apparatus having the configuration shown in Figure **14** was used to measure an action of Carbachol to a neuron, where the neuron was prepared from Lymnaea Stagnalis.

Before and after 50 µM-concentration Carbachol was applied to a Lymnaea Stagnalis neuron, signals were obtained from the measurement section (signal source) **101**, and the standard deviation of the signals was calculated in a manner similar to that of Example 1. The normal distribution approximation section **103** plotted the standard deviations into a graph which is shown in Figure **18**.

Figure **18(A)** shows a histogram of standard deviation values calculated every 5 miliseconds from time-series data of an electrical signal for 10 seconds before applying Carbachol. Figure 18(B) shows a histogram of standard deviation values calculated every 5 miliseconds from time-series data of an electrical signal for 10 seconds after applying Carbachol. As shown in Figures **18(A)** and **(B)**, the histograms before and after applying Carbachol approximated normal distributions. The average and half-width of the resultant normal distribution graph are 0.478 and 0.109, respectively, before the application, and 0.703 and 0.175, respectively, after the application. Thus, it was confirmed that the average of the standard deviation every 5 miliseconds was increased from before to after applying Carbachol. This is because applying Carbachol activates ion channels of a Lymnaea Stagnalis neuron and the opening or closing of the activated ion channel causes a change in action potential.

Figure **19** shows a histogram of standard deviation values every 5 miliseconds where data obtained by a conventional intracellular recording method was subjected to signal processing as described above. As can be seen from Figure **19**, the measurement result of the extracellular recording method is similar to that of the intracellular recording method.

As described above, according to the technique of the present invention, cellular activities associated with the opening or closing of ion channels and changes therein can be measured without conventional intracellular recording methods. Therefore, the present invention makes possible qualitative or quantitative categorization of the action or effect of a drug by measurement of ion channel activities and comparison of the absolute values of or an increase or decrease in ion channel activities before and after applying a drug into a cell or depending on the amount of the drug.

### (Example 5)

Intracellular recording studies and the like have confirmed that the activity of the Ca ion channel of smooth muscle cells is inhibited by nifedipine in a concentration-dependent manner when the cell is stimulated by 10 µM norepinephrine. In data obtained by intracellular recording, nifedipine inhibition is represented and plotted by two variables, i.e., a deviation of the average of the normal distribution of standard deviations from a reference value (relative shift value) and a deviation of the half -width thereof from a reference value (relative spread) in Figure **20**. In Figure **20**, circles indicate nifedipine having a changing concentration (0.03 to 30 µM).

The effect of nifedipine on the Ca ion channel, which was recorded by the intracellular recording method, was used as database to categorize the effects of two drugs A and B for inhibiting the Ca ion channel, which were recorded by the same extracellular recording method as that of Example 4. The ion channel activity of a cell was measured by the same method as that of Example 4 while the concentrations of the drugs A and B were changed in the range of 0.03 to 30 µM. Similar tonifedipine, the resultant effect was represented and plotted by two variables, i.e., a deviation of the average of the normal distribution of standard deviations from a reference value (relative shift) and a deviation of the half-width thereof from a reference value (relative spread) in Figure **20**. In Figure **20**, triangles indicate compound A, while squares indicate compound B. As shown in Figure **20**, compound A (triangle) behaves in a concentration-dependent manner in substantially the same manner as that of nifedipine (circles) within the above-described concentration range. Therefore, compound A was inferred to be a Ca ion channel inhibitor similar to nifedipine. In contrast, compound B has substantially no changes in the above-described relative shift and relative spread, so that compound B is highly probably not a blocker of Ca ion channels which are present in the smooth muscle cell.

As described above, the method of the present invention makes it possible to deduce the effect of an unknown drug. Moreover, for example, drugs may be assessed using a threshold indicated by a dashed circle indicating that the above-described relative shift and relative spread are each within about 5% as shown in Figure **20** (specifically, a concentration giving a measured value plotted within the circle does not have any pharmaceutical effect, while a concentration giving a measured value not plotted within the circle has a pharmaceutical effect), thereby making it possible to screen drugs efficiently.

In the above-described examples, a deviation of the average of a normal distribution from a reference value and a deviation of the half-width thereof from a reference value are employed. Alternatively, corresponding parameters of standard deviation and variance may be used to estimate the effect of a drug.

As described above, in the present invention, the arrangement and environment or characteristics of the reference electrode and the measuring electrode are adapted to characterize a change in electrical characteristics of a biological sample. Therefore, an electrical change in a biological sample can be measured without forming a high resistance seal (gigaseal) between a cell (or a tissue) and a measuring device. Further, in the present invention, the step of detecting a change in electrical characteristics of a biological sample representatively processes a digital signal (predetermined time-series data) captured at a constant sampling rate, so that a significant signal representing the opening or closing of an ion channel can be extracted from noise, and measured and categorized.

Note that the apparatus shown in Figure **13** or **14** is used in the above-described examples, but alternatively, an apparatus shown in Figure **15** or **16** may be used in place of the apparatus of Figure **13** or **14**.

The apparatus shown in Figure **15** comprises a sample number categorization section indicated by reference numeral **111** in addition to the apparatus of Figure **14**.

The apparatus shown in Figure **16** is the same as the apparatus of Figure **14**, except that the apparatus of Figure **16** comprises a plurality of signal sources **101** comprising the sensor substrate of the present invention and further a signal generation section **104** for stimulating the signal sources **101**.

Note that the above-described Examples 1 to 5 only illustrate the usefulness of the apparatus and method of the present invention and are not intended to limit compounds used, oxygen concentrations, and the like.

Although the present invention is described with reference to the above-described examples, the present invention is not limited to the examples. The present invention can be implemented in embodiments having various variations, modifications and changes without departing from the scope and spirit of the present invention.

### INDUSTRIAL APPLICABILITY

By utilizing the device of the present invention, an apparatus capable of electrophysiological evaluation of cells simply and quickly as well as automatically is provided. This apparatus can be applied to drug screening and the like.

The device of the present invention overcomes drawbacks of a conventional patch clamp method which requires a high level of skill or a conventional fluorescent pigment method which includes a number of testing steps and has a poor S/N ratio. With the device of the present invention, it is possible to provide an apparatus capable of screening drug candidate compounds simply and quickly, and dramatically reduce the long time required for conventional drug screening. Further, since such an apparatus does not require a high level of special skill and can automatically collect data and process a signal, anybody can easily measure an extracellular potential.

A measuring method and apparatus which can extract an electrical change associated with the opening or closing of an ion channel of a cell from a signal by extracellular recording, which is conventionally impossible to detect, as a significant signal are provided. By using the simple measuring probe (sensor substrate) not requiring a dedicated control apparatus, a method and apparatus capable of measuring a large amount of biological samples simultaneously in a short time is provided. In the apparatus and method, a chemical substance or a fluorescence substance is not used for measurement, it is not necessary to consider side effects of a chemical substance or changes in fluorescence sensitivity over time. Extracellular recording can provide an indicator for the macro channel activity of an entire cell and can be applied to quick drug screening.

## Claims

1. A device for measuring an extracellular potential, comprising at least one well comprising means for holding a cell provided on a substrate, a measuring electrode for detecting an electrical signal of each of the at least one well, and a reference electrode.

2. A device according to claim 1, wherein the cell holding means comprises at least one depression provided within the well, and has a throughhole at a bottom surface thereof, the throughhole being linked to means for suctioning the cell.

3. A device according to claim 2, wherein the measuring electrode is disposed within a space, the space being in communication with the well via the throughhole.

4. A device according to claim 2, wherein the cell is tightly held by an opening of the depression.

5. A device according to claim 3, wherein the opening of the depression has a diameter of 10 to 50 µm and the throughhole has a diameter of about 2 to 10 µm.

6. A device according to claim 1, wherein the substrate is made of a material selected from the group consisting of a silicon wafer, Teflon, polystyrene and polycarbonate.

7. A device according to claim 1, wherein the substrate is an insulating substrate, and the device further comprises means for suctioning the cell.

8. A device according to claim 1, wherein the well for culturing the cell is made of a material selected from the group consisting of silicone, plastic, SiO₂, and rubber.

9. A device according to claim 1, wherein the reference electrode is in the shape of a ring.

10. A device according to claim 9, wherein the reference electrode is made of a conductive material.

11. A device according to claim 7, wherein the insulating substrate comprises a throughhole having a diameter of 6 µm.

12. A device according to claim 11, wherein the insulating substrate comprises a supporting layer, and the supporting layer is a SOI substrate having a thickness of at least 10 µm.

13. A device according to claim 12, wherein the supporting layer is selected from the group consisting of silicon, plastic, SiO₂, and rubber.

14. A device according to claim 11, wherein the supporting layer has a thickness of 1 µm or more.

15. A device according to claim 7, wherein the cell suctioning means is made of silicone, plastic, SiO₂, and rubber, a thickness of the cell suctioning portion being 10 µm or more.

16. A device according to claim 1, wherein an inner wall of the depression is treated to become hydrophilic.

17. A method for measuring an extracellular potential, comprising the steps of:
providing a reaction system for measuring an electrical characteristic of a biological sample;
disposing an intact cell of interest in the reaction system; and
detecting an electrical characteristic of the cell of interest,
wherein the reaction system comprises at least one well comprising means for holding a cell provided on a substrate, a measuring electrode for detecting an electrical signal of each of the at least one well, and a reference electrode.

18. A method according to claim 17, wherein the electrical characteristic detecting step comprises detecting the electrical characteristic of the cell of interest at least twice, and comparing at least two values of the detected electrical characteristic.

19. A method according to claim 17, wherein a capacity of a region, in which the measuring electrode is provided, is smaller than a capacity of a region, in which the reference electrode is provided.

20. A method according to claim 17, wherein a surface area of the reference electrode is smaller than a surface area of the measuring electrode.

21. A method according to claim 17, wherein an impedance of the measuring electrode is lower than an impedance of the reference electrode.

22. A method according to claim 17, wherein the impedance of the measuring electrode at 10 Hz to 10 kHz is smaller than the impedance of the reference electrode at 10 Hz to 10 kHz.

23. A method according to claim 17, wherein the substrate is an insulating substrate; the cell holding means is a throughhole immersed in an electrolyte and disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an amount of electrolyte immersing the reference electrode is different from an amount of electrolyte immersing the measuring electrode.

24. A method according to claim 23, wherein the amount of electrolyte immersing the reference electrode is 5 times or more as great as the amount of electrolyte immersing the measuring electrode.

25. A method according to claim 17, wherein the substrate is an insulating substrate; the cell holding means is a throughhole disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an electrode area of the reference electrode is different from an electrode area of the measuring electrode.

26. A method according to claim 25, wherein the electrode area of the reference electrode is 1/5 or less of the electrode area of the measuring electrode.

27. A method according to claim 17, wherein the substrate is an insulating substrate; the cell holding means is a throughhole disposed in the insulating substrate; when the cell of interest is disposed on the throughhole, the reference electrode is disposed in the vicinity of a first side of the insulating substrate on which the cell of interest is disposed, the measuring electrode is disposed at a second side of the insulating substrate opposite to the first side, and an impedance of the reference electrode is different from an impedance of the measuring electrode.

28. A method according to claim 27, wherein the impedance of the reference electrode is 5 times or more as great as the impedance of the measuring electrode.

29. A method according to claim 17, wherein the distance of the reference electrode from the cell of interest is longer than the distance of the measuring electrode from the cell of interest.

30. A method according to claim 17, wherein in the extracellular potential measuring device, the area of the reference electrode : the area of the measuring electrode =1:5, the impedance of the reference electrode : the impedance of the measuring electrode = 5:1, or the volume of the electrolyte immersing the reference electrode : the volume of the electrolyte immersing the measuring electrode = 5:1.

31. A method according to claim 17, wherein the extracellular potential is a signal associated with an activation of an ion channel or receptor of a cell, or an action of an intracellular signal transduction system.

32. A method according to claim 17, wherein the electrical characteristic detecting step is performed in the presence of a standard chemical substance having a known action on the cell of interest and in the presence of a subject chemical substance, and the method further comprises the step of:
comparing electrical characteristics obtained in the presence of the standard chemical substance and in the presence of the subject chemical substance, and characterizing an action of the subject chemical substance on the biological sample.

33. A method according to claim 17, wherein the cell of interest disposing step comprises the steps of:
introducing measuring solution into the well;
generating a difference in pressure between the well and an opening of a throughhole opposite to the well, the throughhole being in communication with the well; and
allowing the pressure difference to be a magnitude of 1/10 or less.

34. A method according to claim 33, wherein the pressure difference is generated by reducing the pressure through the opening.

35. A method according to claim 33, wherein the pressure difference is generated by applying pressure to the well.

36. A method according to claim 33, wherein the pressure difference is generated by reducing the pressure through the opening and applying pressure to the well.

37. A method according to claim 33, wherein the pressure difference is 0.01 to 0.5 atm.

38. A quick drug screening apparatus, comprising an extracellular potential measuring device according to claim 1, an electrical signal detecting section linked to an measuring electrode of the extracellular potential measuring device, and means for processing a signal, wherein the signal processing means processes a plurality of electrical signals from the electrical signal detecting section and displays an activity state of a cell.
